# EUROPEAN PATENT SPECIFICATION

(11) **EP 3 930 612 B1**
(45) Date of publication and mention of the grant of the patent: **28.10.2026**
(21) Application number: 20715578.9
(22) Date of filing: 28.02.2020
(51) Int. Cl.: A61B 18/12, A61B 18/14

(54) **BREACH DETECTION IN BIPOLAR ELECTROSURGICAL INSTRUMENT SHEATH**
DURCHBRUCHSDETEKTION IN EINER HÜLLE EINES BIPOLAREN ELEKTROCHIRURGISCHEN INSTRUMENTS
DÉTECTION DE RUPTURE DANS UNE GAINE D'INSTRUMENT ÉLECTROCHIRURGICAL BIPOLAIRE

(30) Priority: 01.03.2019 US 201962812731 P
(43) Date of publication of application: 05.01.2022
(73) Proprietor: Intuitive Surgical Operations, Inc., Sunnyvale, CA 94086 (US)
(72) Inventor: MARION, Duane W., Sunnyvale, California 94086 (US); CLEMENTS, Kyle W., Sunnyvale, California 94086 (US); HAKIM, Zahi P., Sunnyvale, California 94086 (US); RAUCH, Andrew D., Sunnyvale, California 94086 (US); YADLOWSKI, Ann, Sunnyvale, California 94086 (US)
(74) Representative: MacDougall, Alan John Shaw
(86) International application number: PCT/US2020/020413
(87) International publication number: WO 2020/180693

(56) References cited:
- EP-A1- 0 596 909
- EP-A2- 2 881 050
- WO-A1-2018/222899

## Description

### BACKGROUND

Electrosurgery involves the use of electricity to cause thermal destruction of biological tissue to cut or remove the tissue through desiccation, coagulation, or vaporization, for example. Benefits include the ability to make precise cuts with limited blood loss. Electrosurgical instruments are frequently used during surgical procedures to help prevent blood loss in hospital operating rooms or in outpatient procedures. The two types of electrosurgery most commonly used are high-frequency electrosurgery and electrocautery. High-frequency electrosurgery involves high-frequency (radio frequency) alternating current that is converted to heat by resistance as it passes through the tissue. The heat buildup within the tissue causes thermal tissue damage resulting in incision or debulking, for example. Electrocautery also involves direct transference of heat to tissue. Instead of passing electrical current through the tissue, however, the current is used to heat a handheld element, which is then applied to the tissue. Additional modalities of electrosurgery include electrolysis, which uses a chemical reaction created by direct current to damage tissue, and coblation, which uses an electrical current to ionize a conduction medium such as isotonic saline, which is then used to transmit heat to tissue.

Bipolar and monopolar are examples of two modalities for energy delivery during an electro surgical procedure. A monopolar electrosurgical technique involves passage of current from a probe electrode, to patient tissue and through the patient tissue to a return pad to complete the electric current circuit. A bipolar electrosurgical technique typically involves current passage through only a portion of patient tissue disposed between two arms of a forceps shaped electrode. The conductive leads of a bipolar instrument are surrounded by an electrically conductive safety shield that is surrounded by the non-conducting outer insulating sheath.

During electrosurgery it is common for an active monopolar energy instrument to contact an electrode of a nearby bipolar instrument and to transmit energy to the bipolar instrument arms, particularly when the bipolar instrument can also be used for mechanical grasping. This contact can either be inadvertent or intentional, as some surgeons have been taught to "buzz" the bipolar grasper with monopolar energy to create a hemostatic effect, for example. When this occurs, the monopolar energy can travel through the bipolar instrument arms and become capacitively coupled to the shield of the bipolar instrument. If this monopolar energy is coupled to the bipolar instrument and the insulative sheath of the bipolar instrument suffers a breach, an alternate aberrant current path that includes the shield is created. A typical bipolar instrument operates at a voltage in a range of approximately 60Vp-500Vp, and a typical monopolar instrument operates at a voltage in a range of approximately 300Vp-3,000Vp, for example. Thus, such aberrant path of current flow has the potential to create an unintended patient burn injury to patient tissue at a sheath breach location.

Document EP0596909 A1 discloses an electrosurgical apparatus comprising an active electrode probe, a tubular conductive shield having electrical insulation disposed over the outer surface, at least one layer of electrical insulation between the active probe and the shield, and an electrical terminal connected to the shield. The document further discloses monitor circuitry and phase sensing circuitry detecting an in phase relationship as an abnormal condition corresponding to a damage to the electrical insulation.

### SUMMARY

In one aspect a system detects a breach of an insulative sheath in a bipolar electrosurgical instrument. The system includes a first pulse detection circuit to detect a first HF signal component of the HF signal conducted on the lead and includes a second pulse detection circuit to detect a second HF signal component of the HF signal conducted on the shield. Phase difference sampling logic produce sample values indicative of a phase difference between the first HF signal component and the second HF signal component. Current detection logic detects current flow between the shield and anatomical tissue based upon the sample values.

In another aspect, a method is provided to detect a breach of an insulative sheath in a bipolar electrosurgical instrument. The method includes detecting pulses of a first HF signal component of the HF signal conducted on the lead and detecting pulses of a second HF signal component of the HF signal conducted on the shield. Sample values are produced that are indicative of a phase difference between the pulses of the first HF signal component and the second HF signal component. Current flow between the shield and anatomical tissue is detected based upon the sample values.

In yet another aspect a system detects a breach of an insulative sheath in a bipolar electrosurgical instrument. The system includes a first pulse detection circuit to detect a first HF signal component of the HF signal conducted on the lead and includes a second pulse detection circuit to detect a second HF signal component of the HF signal conducted on the shield. Magnitude difference sampling logic produce sample values indicative of a magnitude difference between the first HF signal component and the second HF signal component. Current detection logic detects current flow between the shield and anatomical tissue based upon the sample values.

In another aspect, a method is provided to detect a breach of an insulative sheath in a bipolar electrosurgical instrument. The method includes detecting pulses of a first HF signal component of the HF signal conducted on the lead and detecting pulses of a second HF signal component of the HF signal conducted on the shield. Sample values are produced that are indicative of a magnitude difference between the pulses of the first HF signal component and the second HF signal component. Current flow between the shield and anatomical tissue is detected based upon the sample values.

The scope of the invention is defined by the claims. A more complete understanding of examples of the present invention will be afforded to those skilled in the art, as well as a realization of additional advantages thereof, by a consideration of the following detailed description of one or more examples. Reference will be made to the appended sheets of drawings that will first be described briefly.

### BRIEF DESCRIPTION OF DRAWINGS

Examples of the present invention and their advantages are best understood by referring to the detailed description together with the drawings. It should be appreciated that like reference numerals are used to identify like elements illustrated in one or more of the figures. It should also be appreciated that the figures may not be necessarily drawn to scale.
**Figure 1** is an illustrative schematic diagram showing an example bipolar surgical electrosurgical instrument and an example monopolar surgical electrosurgical instrument selectively coupled to receive electrosurgical signals from an electrosurgical generator unit.
**Figure 2A** is an illustrative axial cross-sectional view of the bipolar leads cable.
**Figure 2B** is an illustrative longitudinal cross-sectional view of the bipolar leads cable with an intact outer sheath.
**Figure 2C** is an illustrative longitudinal cross-sectional view of the bipolar leads cable with a breached outer sheath.
**Figure 3A** is an illustrative physical model representing an electrical contact point between an activated monopolar end effector and the bipolar end effector.
**Figure 3B** is an illustrative circuit model representing the physical model of **Figure 3A****.**
**Figures 4A-4B** are illustrative block diagrams representing an example monitor system coupled to a bipolar ESI to monitor characteristics of a high frequency (HF) signal component parasitically coupled to a shield conductor, during electrical contact between an activated monopolar end effector and a bipolar end effector when an insulative sheath is intact (**Figure 4A**) and when the sheath is breached (**Figure 4B**).
**Figure 5** is an illustrative schematic diagram showing details of a first example monitor circuit that detects RF signal phase shift change events.
**Figure 6** is an illustrative flow diagram representing a monitoring process to monitor a phase relationship between first HF monopolar signal component and a second HF monopolar signal component to detect an occurrence of electrical current flow within patient tissue indicative of a breached insulative sheath.
**Figure 7** is an illustrative signal diagram showing example signals produced by the first example monitor circuit during the process of **Figure 6**.
**Figure 8** is an illustrative schematic diagram showing details of a second example monitor and detection circuit that detects I-IF signal magnitude or phase change events.

### DETAILED DESCRIPTION

### Electrosurgical Instruments

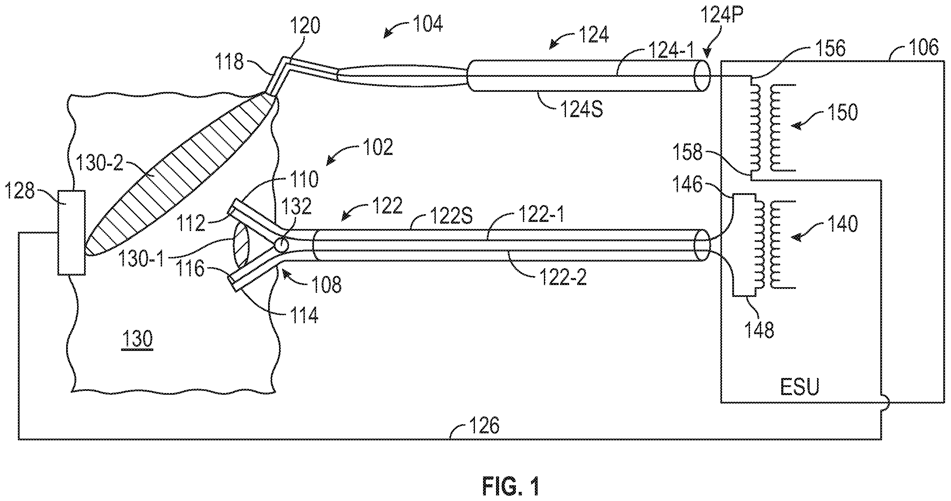

**Figure 1** is an illustrative schematic diagram showing an example bipolar surgical electrosurgical instrument 102 and an example monopolar surgical electrosurgical instrument 104 selectively coupled to receive electrosurgical signals from an electrosurgical generator unit (ESU) 106. An electrosurgical signal typically is a high frequency signal (HF), ordinarily in a radio frequency range (RF) that has a voltage level suitable to achieve a desired surgical effect such as desiccation, coagulation, or vaporization of patient tissue 130, for example. The voltage level of an electrosurgical signal is selected according to the desired surgical effect. The first electrosurgical instrument (ESI) 102 includes a first end effector 108 that includes a first jaw member 110 including a first electrode 112 and a second jaw member 114 that includes a second electrode 116. The second ESI 104 includes a second end effector 118 that includes a single electrode, a monopolar electrode 120. A bipolar leads cable122 includes first and second bipolar leads that extend between the ESU and the bipolar end effector 108. A first bipolar lead within the cable 122 is electrically coupled to the first electrode 112 within the first arm 110 of the bipolar end effector 108. A second bipolar lead within the cable 122 is electrically coupled to the second electrode 116 within the second arm 114 of the bipolar end effector 108. A monopolar lead cable 124 extends between the ESU 106 to the monopolar instrument 104. Monopolar current passes down monopolar electrical lead 124-1 to monopolar electrode 120then through patient tissue 130-2 to a return conductor pad 128 that is placed in contact with patient tissue 130 which provides a return path for monopolar current through 126 back to ESU 106. Commonly assigned U.S. Provisional Patent Application, Serial No. 62/513,287, filed May 31, 2017, entitled Electrosurgical Output Stage with Integrated DC Regulator, describes an ESU 106 in accordance with some embodiments.

The end effector 108 of the bipolar ESI 102 includes an articulated jaw that includes first and second members 110, 114 that articulate relative to one another about a pivot axis 132. At least one of the first and second jaw members 110, 114 is mounted to rotatably pivot about the pivot axis 132 between an open position in which the first and second jaws 110, 114 are spaced apart from each other and a closed position for grasping biological tissue 130-1 between them. The first and second electrodes 112, 116 are mounted upon the members 110, 114 to electrically contact biological tissue 130-1 grasped between the first and second jaw members 110, 114. During normal operation, while the members 110, 114 grip tissue 130-1 between them, the ESU 106 imparts a high frequency electrosurgical signal between the first and second electrodes 112, 116 to cause electrical current to flow through the tissue portion 130-1 grasped between the members 110, 114 to impart heat to the first tissue portion 130-1 to thereby impart an electrosurgical surgical effect such as desiccation, coagulation, or vaporization, for example.

The end effector 118 of the monopolar ESI 104 includes the single third electrode 120 that may be placed in contact with a patient's biological tissue 130-2. During normal operation, the ESU 106 imparts a high frequency electrosurgical signal between the third electrode 120 and the return conductor pad 128 to cause electrical current to flow through a second tissue portion 130-2 disposed between the third electrode 120 and the return conductor pad 128 to cause electrical current to flow through the second tissue portion 130-2 to impart to the second tissue portion 130-2 an electrosurgical effect. The return conductor pad 128 has a surface area that is large enough so that patient tissue in physical contact with the pad has a large enough surface area so that return current to the ESU 106 spreads across a wide enough patient tissue area 130 to limit the current density sufficiently to avoid tissue burns or other trauma due to the return current, for example.

The bipolar ESI 102 ordinarily uses lower voltage, and therefore lower energy, than the monopolar second ESI 104. Because of the lower energy level, bipolar ESI 102 may have a more limited ability to cut and coagulate large bleeding areas and is more ideally used for those procedures where the first biological tissue portion 102 can be easily grabbed on both sides by the members 110, 114 containing the first and second electrodes 112, 116. Thus, in bipolar surgery, the electrosurgical current in the patient is restricted to just the tissue between the electrodes 112, 116, which may provide better control over the area being targeted and help prevent damage to other sensitive tissues.

The bipolar leads cable 122 and the monopolar leads cable 124 span a distance between the ESU 106 and the respective bipolar and monopolar ESIs 102, 104. The bipolar leads cable 122 includes an outer insulative sheath 122S, which may include insulating material that encloses first and second conductive bipolar leads 122-1, 122-2, often referred to as 'electrodes', that extend within it. The monopolar lead cable 124 includes a cable outer sheath 124S, which may include insulating material that encloses a third conductive monopolar lead 124-1 that extends within it.

The ESU 106 includes a first transformer circuit 140 to selectably couple an electrosurgical signal between the first and second bipolar leads 122-1, 122-2, also referred to as active and return leads. The ESU 106 controllably electrically couples and decouples respective first and second terminals 146, 148 of the first transformer 140 to and from the respective first and second bipolar leads 122-1, 122-2. The ESU 106 includes a second transformer circuit 150 to selectably couple an electrosurgical signal between the monopolar lead 124-1 and the third (return) conductor cable 126. More specifically, the ESU 106 is configured to controllably electrically couple and decouple the first and second terminals 156, 158 of the second transformer 150, respectively, to and from the third monopolar lead 124-1 at the proximal end portion 124P of the second cable 124 and the third (return) conductor cable 126.

### Parasitic Capacitance within Bipolar Leads

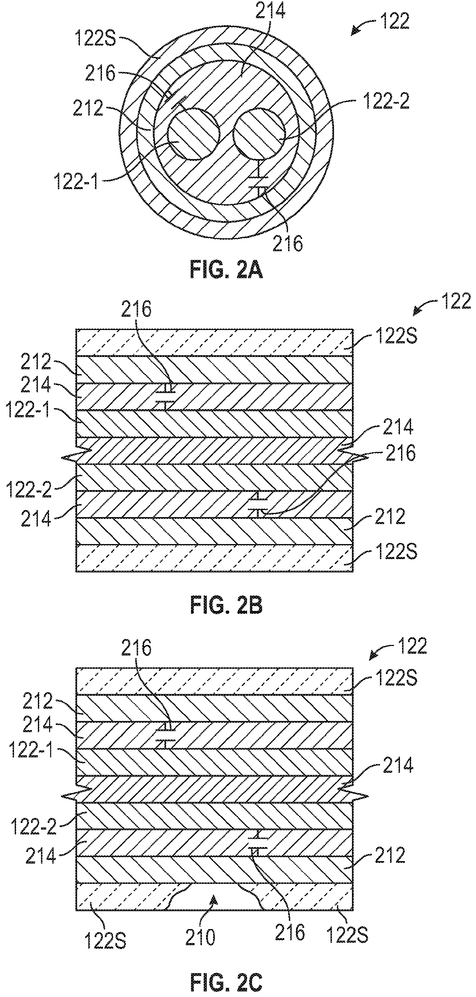

**Figure 2A** is an illustrative axial cross-sectional view of the bipolar leads cable 122. **Figure 2B** is an illustrative longitudinal cross-sectional view of the bipolar leads cable 122 with an intact outer sheath 124S. **Figure 2C** is an illustrative longitudinal cross-sectional view of the bipolar leads cable 122 with a breached 210 outer sheath 122S. The bipolar leads cable 122 includes an elongated tubular shield conductor 212 that is surrounded by the non-conducting tubular outer insulating sheath 122S. The shield 212 surrounds the first and second (active and return) bipolar leads 122-1, 122-2 to provide protection against unintended parasitic capacitive coupling of monopolar electrosurgical energy from the bipolar leads 122-1, 122-2 to the patient, Capacitively coupled energy can potentially lead to electrical arcing or conduction to patient tissue 130 that may cause thermal injury to the patient. Parasitic capacitance 216 between the shield 212 and the bipolar leads 122-1, 122-2, allows the unintended transfer of monopolar energy from ESI 104 to the bipolar leads 122-1, 122-2 and the shield 212. The insulating sheath 122S electrically isolates the capacitively coupled conductor shield 212 from the patient tissue 130. A dielectric material 214 is disposed between the shield 212 and the first and second bipolar leads 122-1, 122-2 and between the first and second bipolar leads themselves. A breach 210 in the insulation sheath 122S such as that shown in Figure 2C, can occur due to unintended or aggressive use during the surgical procedure, such as another instrument (not shown) physically contacting the bipolar leads cable 122 during a surgical procedure, for example.

### Aberrant Current Flow to Due to Breach in Insulative Sheath

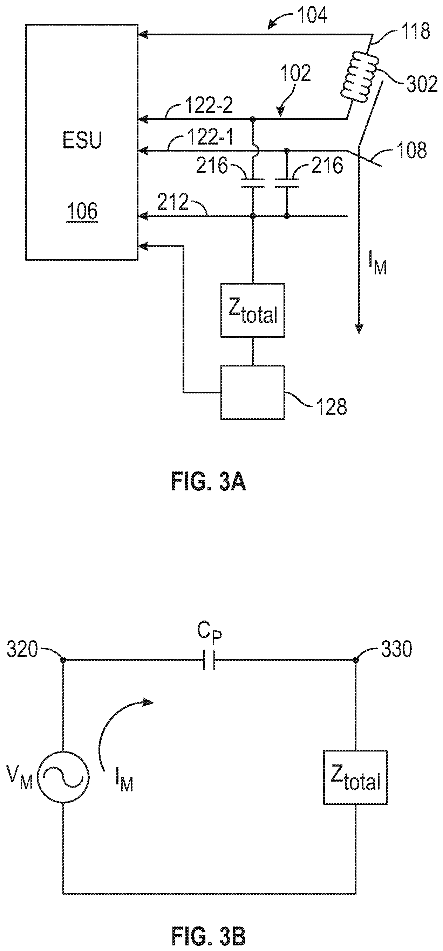

**Figure 3A** is an illustrative physical model representing an electrical contact point 302 between an activated monopolar end effector 118 and the bipolar end effector 108. A high frequency (HF) monopolar current I_{M} imparted to the bipolar instrument leads is parasitically coupled to the protective shield. An impedance Rₜₒₜₐₗ, which includes patient tissue impedance electrically couples the shield to the return pad 128, which is electrically coupled to the ESU (not shown). Thus, aberrant monopolar current I_{M} flows from the monopolar electrode 120 to bipolar electrode 116 (or 112) to one or both of bipolar leads 122-1 and 122-2 to shield 212 to a path having impedance Rₜₒₜₐₗ, to return lead (not shown). In general, monopolar signal components on the two bipolar leads have matching phases and magnitudes. As explained more fully below, if the sheath 122S is intact, Rₜₒₜₐₗ includes impedance of patient tissue (R_{P}) plus impedance of the insulative sheath resistance (Rₛₕ). However, if the sheath 122S is breached, Rₜₒₜₐₗ includes only impedance of patient tissue (R_{P}).

**Figure 3B** is a simplified illustrative circuit model representing the physical model of **Figure 3A****.** the circuit model includes a monopolar voltage source (V_{M}), a parasitic coupling capacitance C_{P}, and a path impedance Rtotal, which includes patient tissue, between the shield and the return pad. A bipolar lead node 320 is coupled between V_{M} and C_{P} A bipolar shield node 130 is coupled between C_{P} and Rtotal. The monopolar current I_{M} flows within the circuit model. When the insulative sheath is breached (i.e. no sheath resistance), Rtotal arises primarily due to patient resistance which is primarily resistive and typically measures in a range oftens to thousands of ohms. When the insulative sheath is intact Rtotal arises primarily due to sheath resistance which is which typically measures in a range of about 1M ohm or greater. ${Z}_{equivalent} = {R}_{total} - j * {X}_{C}$

Z_{equivalent} is the total impedance of the branch: the impedance arising from the combination of Xc and Rtotal. When the sheath is intact Rtotal includes the sheath (Rsh) and patient (Rp) resistance in overall impedance of the branch. In this case the resistance of the branch is much larger than the reactance, since Rtotal >> Xc when sheath is intact. As such the branch is much more resistive than reactive and the current through the branch leads the voltage driving the current through the branch by a small amount. This leads to a small phase shift in voltage between 320 and 330. 320 is the bipolar electrode and 330 is the bipolar shield. These are the electrical points at which we measure phase shift.

When a sheath breach occurs, implying the shaft of the bipolar instrument comes into direct contact with the patient, the sheath resistance, Rsh, is not included in Rtotal of the model. When this happens, the resistance is on the same order of magnitude as the reactance and the overall impedance of the branch becomes more reactive than when an intact sheath is present. The current through the branch now leads the driving voltage by a larger amount than when an intact sheath is present. The leading shift in current through the branch creates a larger phase shift in voltages between 320 and 330. These are the electrical points at which we measure phase shift. We set a phase shift threshold to guarantee that if crossed we must have a sheath breach. It will be appreciated that Rₜₒₜₐₗ plays a role in phase shift between an I_{M} current component at the bipolar lead node 320 and parasitically coupled I_{M} current component at the shield node 330. When Rtotal is large (i.e. intact sheath), then Rtotal >> Zc and a phase shift, θ_{zequ}, between bipolar and sheath nodes 320, 330 is smaller. When Rtotal is small (i.e. breached sheath), the phase shift θzequ is larger and a phase shift between the nodes 320, 330 is larger.

### Monitor and Detection Circuit Coupled to Detect Change in Characteristic of HF Signal Coupled to Shield by Parasitic Coupling Capacitance

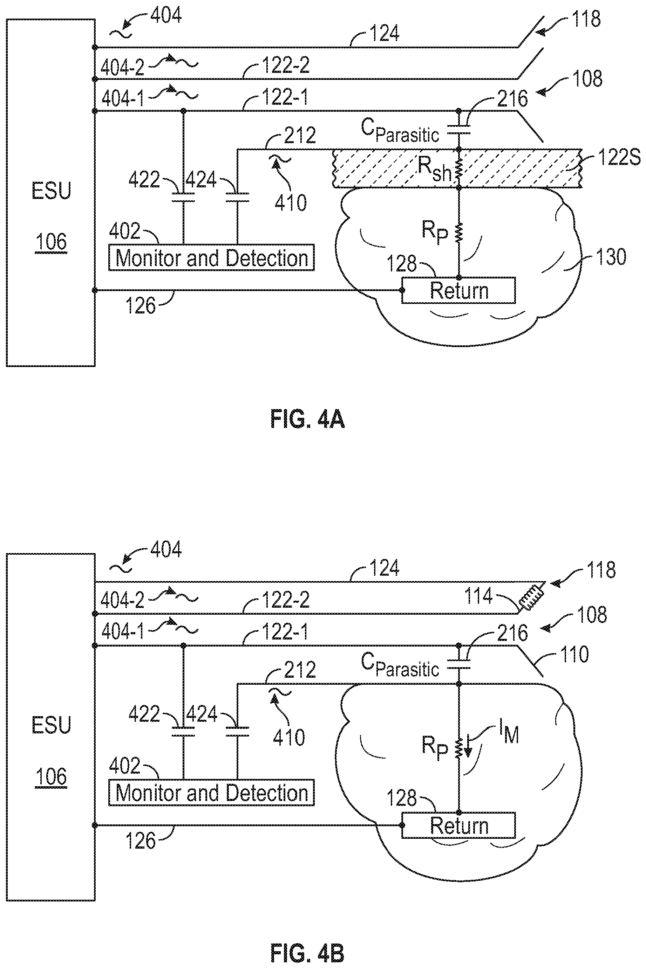

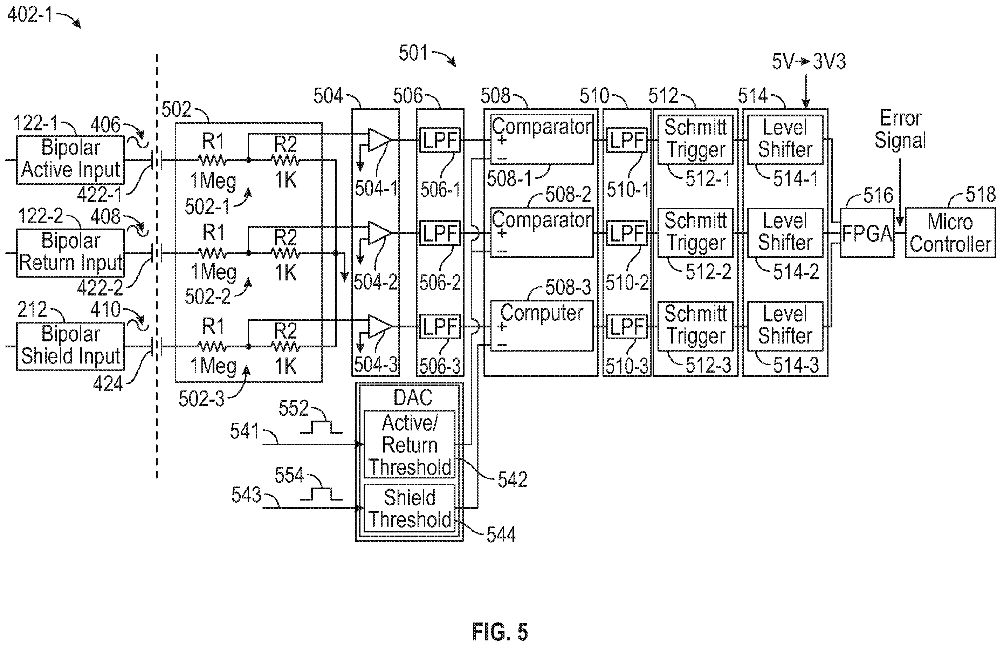

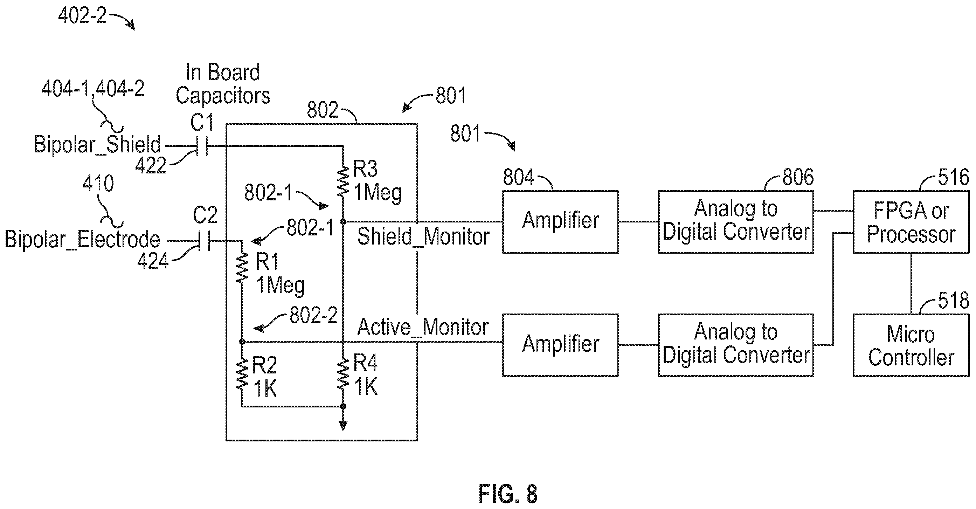

**Figures 4A-4B** are illustrative block diagrams representing an example monitor and detection system 402 coupled to a bipolar lead 122-1 (or 122-2) to monitor characteristics of an HF signal parasitically coupled to a shield conductor 212 to detect electrical discharge current between an activated monopolar end effector 118 and a bipolar end effector 108. **Figure 4A** shows electrical characteristics of a current path between the shield conductor 212 and the monopolar instrument return pad 128 when an insulative sheath 122S is intact. **Figure 4B** shows electrical characteristics of a current path between the shield conductor 212 and the monopolar instrument return pad 128 when the insulative sheath 122S is breached. The current path is displaced from bipolar end effector 108. More particularly, the current path includes a portion of the shield 212 that ordinarily is electrically isolated from patient tissue 130 by the insulative sheath 122S. As explained below, the intact the insulating sheath 122S shown in **Figure 4A** prevents the parasitic capacitive coupling of current from the conductive shield 212 to patient tissue 130. However, the breached insulating sheath 1225 shown in **Figure 4B****,** permits parasitic capacitive coupling of current from the conductive shield 212 to patient tissue 130. The monitor and detection circuit 402 detects potentially dangerous discharge of electrical energy to patient tissue 130, during electrical contact between an activated monopolar end effector 118 and a bipolar end effector 108. More particularly, a first example monitor and detection circuit 402 described below with reference to **Figure 5****,** detects a change in current phase at the shield 212 caused by formation of a series impedance divider due to a breach in the sheath 122S in which the parasitic coupling capacitance 216 and the patient impedance Z_{P} comprise two impedance elements of the series impedance divider, Moreover, a second example monitor and detection circuit 402 described below with reference to **Figure 8****,** detects a change difference in voltage magnitude between the shield 212 and the bipolar electrical lead 122-1. When the sheath is intact Rsh is present and the voltage difference between 122-1 and 212 is small. When the sheath is breached and the shield comes into contact with the patient tissue, only Rp is present, and the voltage difference between 122-1 and 212 is larger than with an intact sheath. A threshold is set in the middle of these two differences. If difference in voltage is larger than a set threshold monopolar energy is disabled.

Referring to **Figures 4A-4B**, the parasitic coupling capacitance (C_{parasitic}) 216 typically is in a range of approximately 120pF, which approximately 3800 ohms (Reactance). The insulative sheath resistance (Rₛₕ) typically is in a range of about 1M ohm to about 1G ohm (Resistive). The patient resistance (R_{P}) is typically in a range of tens to thousands of ohms (Resistive). Referring to **Figure 4A****,** with an intact insulative sheath 1228S very little current can be parasitically coupled to the patient tissue 130 due to the large sheath resistance (Rₛₕ) coupled in series between the parasitic capacitance (C_{parasitic}) 216 and the patient resistance (R_{P}). Referring to **Figure 4B**, however, with a breached insulative sheath, a larger current can be parasitically coupled to the patient tissue 130 due to the parasitic capacitance (C_{parasitic}) 216 being directly coupled in series to the patient resistance (R_{P}).Reffering to both **Figures 4A-4B**, ESU 106 produces an HF monopolar signal 404 on a circuit that includes the monopolar lead 124 between the ESU 106 and the monopolar end effector 118 and includes a path within patient tissue 130 between the monopolar end effector 118 and the return conductor pad 128, and that follows a path on conductor line 126 between the return conductor pad 128 and the ESU 106. During normal operation, the monopolar signal 404 conducted within the monopolar instrument 104 is isolated from the bipolar instrument 102. However, when the activated monopolar end effector 118 electrically contacts the bipolar end effector 108, the monopolar HF signal 404 is coupled to bipolar line 122-1, indicated by a first monopolar signal component 404-1, and is coupled to bipolar line 122-2, indicated by another first monopolar signal component 404-2, Thus, the electrical contact between the activated monopolar instrument and the bipolar instrument results in flow of monopolar signal current on the bipolar leads.

Still referring to both **Figures 4A-4B****,** a first coupling capacitor 422 couples to the monitor and detection circuit 402 a monopolar signal component 404-1 (or 404-2) coupled to the bipolar lead 122-1 (or 122-2). A second coupling capacitor 424 couples to the monitor circuit 402, a second high frequency (HF) monopolar shield signal component 410 coupled by the parasitic coupling capacitor 216 from one of the bipolar leads 122-1, 122-2 to the conductive shield 212. It will be appreciated that the coupled monopolar signal components 404-1, 404-2 have matching phases and magnitudes. It also will be appreciated that the second HF monopolar shield component signal 410 comprises a parasitically coupled portion of at least one of the monopolar signal components 404-1, 404-2 coupled to the bipolar leads 122-1, 122-2. 111e monitor and detection circuit 402 determines whether a characteristic of the second HF monopolar shield signal component 410 coupled by the parasitic coupling capacitor 216 to the conductive shield 212 indicates aberrant current In due to a breach of the protective sheath 210. Aberrant current flow at a breach location causes a change in a characteristic of the portion of the HF shield signal component 410 coupled from one or both of the bipolar leads 122-1, 122-2 to the conductive shield 212 by the parasitic coupling capacitor 216.

### Monitor and Detection Circuit Configured to Detect Change in Phase Shift Characteristic of HF Signal Coupled to Shield by Parasitic Coupling Capacitance

**Figure 5** is an illustrative schematic diagram showing details of a first example monitor and detection circuit 402 that detects RF signal phase shift change events. The inventors realized that the patient tissue in which the aberrant monopolar current I_{M} flows has an impedance that influences characteristics of an RF signal coupled to the shield 212 by the parasitic coupling capacitance 216 between the first and second bipolar leads 122-1, 122-2 and the shield 212. In particular, a phase shift arises from an impedance divider formed by the parasitic coupling capacitance 216 and an impedance Z_{P} that includes patient tissue impedance, and that also includes an insulative sheath impedance if the sheath is intact and not breached.

Coupling capacitors 422-1, 422-2 couple the first HF monopolar signal components 404-1, 404-2 to the first example monitor and detection circuit 402. Coupling capacitor 424 couples the second HF monopolar shield signal component 410 to the first example monitor circuit 402. The coupling capacitors 422-1, 422-2, and 424 each includes a high impedance PCB embedded parallel plate capacitor.

The first example circuit 402 includes pre-processing circuitry 501 that includes an input voltage divider circuit block 502, an input amplifier circuit block 504, a first low pass filter block 506, a comparator circuit block 508, a second low pass filter (LPF) block 510, a Schmidt trigger circuit block 512, and a voltage level shifting circuit block 514. Output of the pre-processing circuitry 501 is provided to a first example logic circuit block 516, which determines a change in a phase shift characteristic of the second HF monopolar signal component 410 coupled to the conductive shield 212 in relation to the first HF monopolar signal component(s) 404-1, 404-2 coupled to the bipolar lead(s) 122-1, 122-2. In the first example monitor and detection circuit 402, the first example logic circuit 516 includes a field programmable gate array (FPGA). A processor circuit 518 produces control signals based upon the phase shift characteristic determined by the monitor and detection circuit 402.

In the first example monitor and detection circuit 402, the detected characteristic is a phase relationship between HF monopolar signal components 404-1, 404-2 on one of the bipolar leads 122-1, 122-2 and the HF monopolar signal component 410 coupled to the conductive shield 212. During activation of a monopolar ESI 104, if the monopolar electrode 118 comes into contact with either bipolar jaws 110, 114, the first example monitor and detection circuit 402 measures a phase difference between the HF monolithic signal compoents 404-1, 404-2 on the bipolar lead(s) 122-1, 122-2 and the HF monopolar signal component 410 capacitively coupled to the bipolar shield 212. If there is no breach in the sheath, then there is negligible phase difference between the signal components. However, if a breach 210 ocurrs in the protective sheath 122S and the protective shield 212 comes into contact with patient tissue 130, an aberrant monololithic current I_{M} flow across the parasitic coupling capacitance 216 causes a considerable phase shift between an HF monopolar signals present on the bipolar leads 122-1, 122-2 and an HF monopolar signal present on the shield 212. The first example monitor and detection circuit 402 measures this phase shift difference. Size and duration of the phase shift is used to determine breachoccurrence. For example, a phase shift difference greater than a specified threshold indicates a tear in the insulative sheath. In the first example monitor and detection circuit, 402 the processor 518 generates control signals based upon the determined size and duration of a measured phase shift. The control signals cause one or more of terminating monopolar energy delivery to prevent patient delivery, sending a message to a user (e.g., a surgeon) or saving data to a storage device for later analysis, for example.

The first example monitor and detection circuit 402 includes an input signal pulse detection channel for each received HF monopolar signal component. A first pulse detection circuit channel pre-processes the HF monopolar signal component 404-1 on the bipolar lead 122-1 for input to the first example logic circuit 516. A second pulse detection circuit channel pre-processes the HF monopolar signal component 404-2 on the bipolar return lead 122-2 for input to the first example logic circuit 516. A third pulse detection circuit channel pre-processes the HF monopolar signal component 410 coupled to the conductive shield 212 for input to the first example logic circuit 516.

Referring to the first pulse detection circuit channel, a first coupling capacitor 422-1 couples the HF monopolar signal component 404-1 from the active bipolar lead 122-1 to the input voltage divider block 502, where a first divider circuit 502-1, which includes resistors R₁, R₂ coupled as shown, divides a voltage level of the HF monopolar signal component 404-1 received on the first channel to a voltage level suitable for input to the voltage amplification block 504, which includes a first amplifier circuit 504-1 to amplify the voltage level of the received first channel RF signal. A first low pass filter 506-1 within the first low pass filter block 506 low pass filters the amplified first channel HF signal. A first comparator circuit 502-1 within the comparator block 508 compares the low pass filtered first channel HF signal with a bipolar lead, threshold detection signal to convert the first channel HF signal to a square wave signal. A first digital-to-analog converter (DAC) circuit 542 converts a provided first digital signal value 552 on line 541 to generate a first (bipolar lead) threshold detection signal. A typical received first channel RF signal has a sinusoidal waveform, and each pulse consists of one-half of a sinusoidal signal cycle. The first comparator circuit 508-1 converts the received first channel RF signal waveform to a square waveform based upon comparison of rising and falling edges of the sinusoidal signal with the shield threshold detection signal. A first low pass filter 510-1 within the second LPF block 510 low pass filters the first channel signal, which has been converted to a square wave waveform. It will be appreciated that the first LPFs 506 filter the raw incoming signal and the second LPFs 510 filter noise of the digital signal output from the comparators 508. A first Schmidt trigger circuit 512-1 within the Schmidt trigger block 512 acts as a noise filter to remove noise from the filtered first channel square wave signal. A first level shifting circuit 514-2 within the level shifting block 514 shifts a voltage level of the smoothed first channel HF square wave signal to a voltage level suitable for input to the first example logic circuit 516. Thus, it will be appreciated that the comparator and the Schmidt trigger cooperate to convert the first HF monopolar signal component 404-1 to a substantially noise-free square wave signal.

The second and third pulse detection circuit channels of the first example monitor and detection circuit 402 pre-process the respective HF monopolar signal component 404-2 on the bipolar lead 122-2 and the HF monopolar signal component 410 on the shield 212 for input to the first example logic circuit 516. The pre-processing of the HF monopolar signal components 404-2, 410 is similar to the pre-processing of the HF monopolar signal component 404-1 except that a third comparator 508-3 compares the HF monopolar shield component signal 410 with second active/return threshold signal produced by a second DAC 544, which converts a provided second digital signal value 554 on line 543 to generate a second (shield) threshold detection signal. Operation of circuit components in the second and third channels and pre-processing of the second and third channel RF signals on the second and third channels will be readily understood by persons of ordinary skill in the art by references to the drawings and the above description of first channel pre-processing. Therefore, in the interest of conciseness, second and third channel pre-processing will not be described in detail herein.

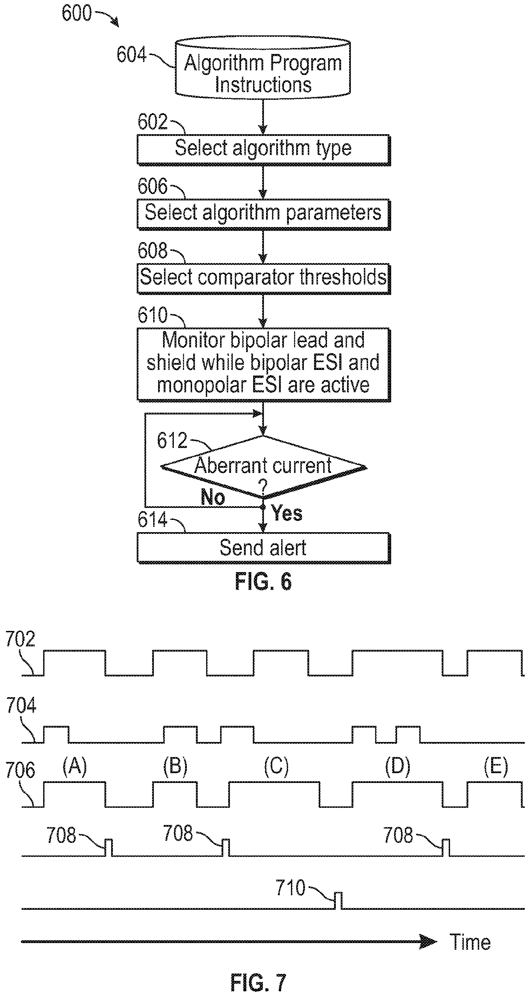

**Figure 6** is an illustrative flow diagram representing a monitoring process 600 to monitor a phase relationship between HF monopolar signal components 404-1, 404-2 and an HF monopolar shield signal component 410 to detect an occurrence of aberrant monopolar current flow In within patient tissue 130 indicative of a breached insulative sheath 122S. The first example logic circuit 516 is configured to perform the first monitoring process of **Figure 6****.** A user provides an input command to the first input logic module 602 to select an algorithm from a storage memory device 604 to configure the logic circuit 516. A storage memory device 604 stores multiple different selectable program instruction to configure the logic device 516 to implement multiple different selectable algorithms, described below, to detect an occurrence of aberrant current flow. A user provides an input command to a second input logic module 606 to input parameters for the selected algorithm. Example input parameters include length of sampling window and phase threshold. A user provides input commands to a third input logic module 608 to configure the logic circuit 516 to select comparator thresholds. More particularly, the user provides an input command to cause provision of the first digital signal 552 to the first DAC 542 to set the shield signal threshold detection level for the first and second comparator 508-1, 508-2. The user provides an input command to cause provision of the second logic signal 554 to second DAC 544 to set the threshold detection signal for the third comparator 508-3. The comparator threshold signals are set to cause the comparators to generate digital outputs ᵢn response to sinusoidal input signals crossing selected thresholds. Different comparator threshold settings can be used for the HF monopolar signal componsnts 404-1, 404-2 on the one hand and for the HF monopolar shield signal component 410 on the other because there can be a difference in voltage amplitude introduced due to the parasitic coupling capacitance. A fourth phase difference sampling logic module 610 monitors a phase relationship between an HF signal 40-4-1, 404-2 coupled from a bipolar lead 122-1, 122-2 and an HF monopolar signal component 410 coupled from the shield 212. A fifth phase shift aberrant current detection logic 612 configures the first example logic circuit 516 to evaluate the phase relationship between the coupled monopolar signal component 404-1, 404-2 and the coupled HF shield signal component 410 based upon the selected algorithm and the selected comparator thresholds to determine whether there has been an occurrence of an aberrant monopolar current I_{M} within patient anatomy 130 due to contact between the two ESIs in concert with a breach in the insulative sheath. A control loop including the fourth and fifth logic modules 610, 612 continues to run while the bipolar and monopolar instruments 102, 104 are active unless the fifth module detects 612 an aberrant current. A sixth alerts logic module 614 configures the first example logic circuit 516 to send a control signal to the processor 518 in response to a detection of an aberrant current flow. As explained above, in response to receiving a signal indicating an occurrence of an aberrant current, the processor 518 provides one or more control signals to alert the processor 518 to cause one or more of terminating monopolar energy delivery to prevent patient delivery, sending a message to a user (e.g., a surgeon) or saving data to a storage device for later analysis, for example.

**Figure 7** is an illustrative signal diagram showing example signals produced by the first example monitor circuit during the process of **Figure 6****.** RF bipolar square wave signals and the HF shield square wave signals produced by the first, second, and third pulse detection circuit channels are received as input at the logic circuit 516. The HF bipolar square wave signal 702 shown in **Figure 7** represents a pre-processed version of one of the HF monopolar signal components 404-1, 404-2 coupled from a bipolar lead 122-1, 122-2 that has been conditioned by one of the first and second pulse detection circuit channels of the pre-processing circuitry 501. The HF shield square wave signal 704 represents a version of an HF monopolar shield signal component 410 coupled from the shield 212 that has been conditioned by the third pulse detection circuit channel of the pre-processing circuitry 501. A pulse sampling window signal 706 is produced by internal logic of logic circuit 516 and represents a sequence of sampling time intervals in which the logic circuit 516 determines a sequence of phase relationships between pulses of the HF square wave signal 702 and pulses of the HF shield square wave signal 704. During successive sampling windows, the logic circuit 516 produces a good phase event sample pulse signal 708 in response to a determination that the HF bipolar lead and HF shield square wave signals have substantially matching phases, and the logic circuit 516 produces a bad phase event sample pulse signal 710 in response to a determination that the RF bipolar and RF shield square wave signals have non-matching phases. Referring to **Figures 6-7****,** the fourth phase difference sampling logic module 610 represented in **Figure 6** configures the logic circuit 516 to identify the occurrence times of centers of successive pulses of the HF bipolar square wave 702 by identifying the times of occurrence of their respective rising and falling edges. Similarly, the fourth phase difference sampling logic module 610 configures the logic circuit 516 to identify the occurrence times of centers of successive pulses of the HF shield square wave 704 by identifying the times of occurrence of their respective rising and falling edges. The fourth phase difference sampling logic module 610 configures the logic circuit 516 to evaluate the phase relationship of HF monopolar signal components 404-1, 404-2 on bipolar leads 122-1, 122-2 and HF monopolar shield signal components on the shield 212 during the successive sampling time windows based upon a measure of difference in occurrence times of square wave pulses corresponding to monopolar signal components on bipolar leads 122-1, 122-2 and square wave pulses corresponding to monopolar signal components on the shield 212 during the successive sampling time windows. For example, during each time window, the logic circuit 516 determines a difference in time of occurrence of a center of a pulse corresponding to a monopolar signal component 404-1, 404-2 on a bipolar lead 122-1, 122-2 and time of occurrence of a center of a pulse corresponding to a monopolar shield signal component 410 on the shield 212 during the time window. The fourth phase difference sampling logic module 610 configures the logic circuit 516 to produce a good phase event signal pulse 708, indicating no occurrence of an aberrant current event, for each sampling time interval in which the time difference between pulse centers is less than a threshold time difference. The fourth phase difference sampling logic module 610 configures the logic circuit 516 to produce a bad phase event signal pulse 710, indicating a possible occurrence of an aberrant current event, for each sampling time interval in which the time difference between centers of the pulses is greater than or equal to a threshold time difference. It is noted that the center points of the pulses are used for the phase shift determination since the widths of the square wave pulses is dependent upon the amplitude of the sine wave input, which varies with signal amplitude.

More particularly, for example, the illustrative signal diagram of **Figure 7** includes five sampling windows: (A)-(E). An example fourth phase difference sampling logic module 610 configures the logic circuit 516 to open a pulse sampling time window upon an occurrence of a leading edge of either of the HF bipolar square wave pulse 702 the HF shield square wave pulse 704. During the first example sampling window (A), a HF bipolar square wave pulse 702 and a HF shield square wave pulse 704 both occur within the threshold time difference, and therefore, a good phase event signal pulse 708 is produced. Similarly, during the second example sampling window (B), a HF bipolar square wave pulse 702 and a HF shield square wave pulse 704 both occur within the threshold time difference, and therefore, a good phase event signal pulse 708 is produced. In the illustrative example signal diagram, the second sampling window (B) closes early, after falling edge occurrences of both the pulse 702 and the pulse 704. As shown in the third example sampling window (C), if a sampling window opens while either a pulse 702 or a pulse 704 is received (e.g., is high), then the time at which the sampling window opens is considered to be the leading edge for that pulse. During the third example sampling window (C), the pulse 702 the pulse 704 are spaced in time by an amount greater than the threshold time difference, and therefore, a bad phase event signal pulse 710 is produced. An example fourth phase difference sampling logic module 610 is configured to handle a situation in which, for example, multiple pulses 702 or multiple pulses 704 occur during a sampling window, as shown in the fourth example sampling window (D). An example sampling logic module 610 can be configured to make a good/bad determination based upon the first pulse, the last pulse or the largest pulse or based upon the midpoint of the first rising edge and the last falling edge, for example. A determination is made for the fourth example sampling window (D) that the pulse 702 and the pulse 704 both occur within the phase difference threshold, and therefore a good phase event sample pulse signal 708 is produced. An example sampling logic module 610 can be configured such that during a sampling window when only one of two pulses 702, 704 occurs, neither a good pulse nor a bad pulse is produced. For example, during the fifth example sampling window (E), there is an occurrence of the pulse 702, but there is no occurrence of the pulse 704, and therefore, no good/bad pulse is produced.

The fifth aberrant current detection logic 612 of Figure 6 configures the logic circuit 516 to use a selected phase-difference based algorithm configured according to selected algorithm parameters to detect aberrant current based upon an evaluation of a HF phase difference between monopolar signal components on the bipolar leads and monopolar signal components on the shield as indicated by a succession of good and/or bad pulses.

### Algorithms to Detect Aberrant Current Based Upon Comparison of Component of an HF Signal Conducted on a Lead with a Component of the HF Signal Conducted on a Shield

A first example selectable phase-difference based algorithm configures the logic circuit 516 to detect an occurrence of aberrant current in response to a continuous sequence of bad phase event sample pulses for at least a prescribed time duration. Selectable parameters for the first example phase-difference based algorithm include a phase difference threshold and the prescribed time duration. An example time duration typically is in a range one-half to three-fourths of the ESU period.

A second example selectable phase-difference based algorithm configures the logic circuit 516 to detect an occurrence of aberrant current in response to at least a prescribed number of bad phase event sample pulses (pulse count) during a prescribed time duration. Selectable parameters for the second example phase-difference based algorithm include a typical phase difference threshold of 30-60 degrees phase shift, and the prescribed bad phase event pulse count and the prescribed time duration. An example time duration typically is in a range five hundred micro-seconds to two milliseconds.

A third example selectable phase-difference based algorithm configures the logic circuit 516 to detect an occurrence of aberrant current in response to at least a prescribed number (count) of consecutive bad phase event sample pulses. Selectable parameters for the third example phase-difference based algorithm include a phase difference threshold and the prescribed consecutive count of bad phase event sample pulses. An example number of consecutive bad phase event sample pulses typically is in a range three to seven.

A fourth example selectable phase-difference based algorithm configures the logic circuit 516 to detect an occurrence of aberrant current in response to at least a prescribed number (count) of consecutive bad phase event sample pulses within a prescribed time duration. Selectable parameters for the fourth example phase-difference based algorithm include a phase difference threshold and the prescribed number of bad phase event pulses and the prescribed time duration.

A fifth example selectable phase-difference based algorithm configures the logic circuit 516 to detect an occurrence of aberrant current in response to observation of a prescribed ratio of bad phase event sample pulses to good phase event sample pulses over a prescribed time duration. Selectable parameter for the fifth example phase-difference based algorithm include a phase difference threshold and the prescribed number ratio and the prescribed time duration.

The ESU 106 produces different modes of bipolar lead signals for different surgical procedures. For example, the ESU 106 produces cut mode signals to cut tissue. Cut mode signals typically are comprised of a continuous (non-pulsed) output. The ESU 106 produces coagulate mode signals to coagulate tissue. Coagulate mode signals typically are pulsed at a very low duty cycle.). The ESU 106 produces blend mode signals to blend tissue. Blend mode signals typically have a duty cycle between continuous and the low duty cycle used for Coagulate.

An algorithm type can be selected using the first input module 602 based upon the monopolar output settings and the ESU mode. For example, the algorithm type may be selected based upon power or effect setting and mode (which implies duty cycle). Similarly, parameters can be selected using the second input module 604 based upon the monopolar output settings and the ESU mode such as power or effect setting and mode (which implies duty cycle).) Comparator thresholds can be selected using the third input module based upon the monopolar output settings and ESU mode. For example, the comparator thresholds can be selected based upon expected amplitude of HF output signals.

### Monitor and Detection Circuit Configured to Detect Change in Magnitude Characteristic of RF Signal Coupled to Shield by Parasitic Coupling Capacitance

**Figure 8** is an illustrative schematic diagram showing details of a second example monitor and detection circuit 402-2 that detects RF signal magnitude change events. As explained above, the inventors realized that the patient tissue in which the aberrant monopolar current I_{M} flows has an impedance Z_{P} that influences characteristics of an HF monopolar signal component coupled to the shield by the parasitic coupling capacitance 216 between the first and second bipolar leads 122-1, 122-2 and the shield 212. In particular, magnitude of monopolar signal component on the shield changes with changes in the series impedance divider Z_{P}, which includes patient tissue resistance (R_{P}) in series with sheath resistance (Rₛₕ), during aberrant monopolar current flow. Referring to equations, for example, as Z_{P} approaches infinity, the monopolar shield voltage approaches the monopolar voltage on the bipolar leads. Thus, if the shield is intact, Z_{P} is effectively infinite, no current flows through patient tissue 130 and a magnitude of the monopolar signal shield voltage matches a magnitude the monopolar signal bipolar lead voltage. However, if the shield is breached, Z_{P} has a lower voltage magnitude, some monopolar signal component current flows through patient tissue 130, and a magnitude of the monopolar signal shield voltage is less than a magnitude of a voltage of the monopolar signal component on the bipolar leads. The second monitor and detection circuit 402-2 is configured to compare magnitude of at least one of the HF monopolar signal components 404-1, 404-2 with magnitude of HF monopolar shield signal component 410 to determine whether or not a potentially harmful monopolar current is flowing through patient tissue. If the magnitudes match, then a determination is made that there is no dangerous current flow. If the magnitudes differ by some prescribed magnitude, then a determination is made that there is a dangerous current flow. The second example monitor and detection circuit 402 includes pre-processing circuitry 801 that includes an input voltage divider circuit block 802, an input amplifier circuit block 804, and an analog to digital converter circuit block 806, which detects signal pulses. An output of the pre-processing circuitry 801 is provided to a logic circuit 516, which determines a change in a signal magnitude characteristic of the HF monopolar shield signal component 410 in relation to the HF monopolar signal components 404-1, 404-2. In the second example monitor circuit, the logic circuit 516 includes an FPGA. A processor circuit 518 produces control signals based upon the change in an RF shield signal magnitude characteristic determined by the second example monitor and detection circuit 402.

More specifically, the second example monitor circuit 402 includes first channel to pre-process one of the HF monopolar signal components 404-1, 404-2 and includes a second channel to pre-process the HF monopolar shield signal component 410. In most cases voltage magnitude is the same on both bipolar leads, and therefore, there is no need to measure voltage on both of them. Referring to the first channel, a first coupling capacitor 422 couples an HF monopolar signal component 404-1 (or 404-2) to the input voltage divider block, where a first divider circuit 802-1, which includes resistors R₁, R₂ coupled as shown, divide a voltage level of the HF monopolar signal component received on the first channel to a voltage level suitable for input to the voltage amplification block 804, which includes a first amplifier circuit (not shown) to amplify the voltage level of the received HF monopolar signal component. A first analog to digital converter (ADC) converts the amplified HF monopolar signal component and provides the amplified RF bipolar lead signal as input to the logic circuit 516.

The second channel of the preprocessing circuit 801 pre-processes the HF monopolar shield signal component 410. The pre-processing of HF monopolar shield signal component 410 coupled from the second coupling capacitor 424 is similar to the pre-processing of the HF monopolar signal component 404-1. Operation of circuit components in the second channel will be readily understood by persons of ordinary skill in the art by references to the drawings and the above description of first channel pre-processing. Therefore, in the interest of conciseness, second channel pre-processing will not be described in detail herein.

A monitoring process performed using the second example monitor and detection circuit 402-2 is similar to that described above with reference to **Figure 6** but without the select comparator block 608. Moreover, in the second monitor and detection circuit 402-2, the logic circuit 516 compares a voltage magnitude of a first monopolar signal component 404-1, 404-2 on at least one of the bipolar leads 122-1 or 122-2 with a voltage magnitude 410 of a second monopolar signal component on the shield 212 to detect potentially dangerous aberrant monopolar current flow. Furthermore, the logic circuit 516 of the second monitor and detection circuit 402-2 is configurable to perform the first through sixth example algorithms modified to compare signal component magnitude differences rather than signal component phase differences. More particularly, an example logic circuit 516 determines whether a magnitude of a difference between the voltage magnitude of a first monopolar signal component 404-1, 404-2 on at least one of the bipolar leads 122-1 or 122-2 with a voltage magnitude 410 of a second monopolar signal component on the shield 212 crosses a threshold magnitude difference. The logic circuit 516 of the example circuit 402-2 produces a good magnitude event sample pulse signal (not shown) in response to a determination that the HF bipolar lead and HF shield signals have substantially matching magnitudes; that is, the magnitude difference does not cross the magnitude difference threshold. The logic circuit 516 of the example circuit 402-2 produces a bad magnitude event sample pulse signal (not shown) in response to a determination that the HF bipolar lead and HF shield signals have non-matching magnitudes; that is the magnitude difference crosses the magnitude difference threshold.

The above description is presented to enable any person skilled in the art to create and use a system and method detect a breach in an insulative sheath of a bipolar electrosurgical instrument. Various modifications to the examples will be clear to those skilled in the art, and the generic principles defined herein may be applied to other examples and applications without departing from the scope of the invention. In the preceding description, numerous details are set forth for explanation. However, one of ordinary skill in the art will realize that the circuitry might be practiced without the use of these specific details. In other instances, well-known circuits and processes are shown in block diagram form in order not to obscure the description of the invention with unnecessary detail. Identical reference numerals may be used to represent different views of the same or similar item in different drawings and in the specification. Thus, the foregoing description and drawings of examples in accordance with the present invention are merely illustrative of the principles of the invention. Therefore, it will be understood that various modifications can be made to the examples by those skilled in the art without departing from the scope of the invention, which is defined in the appended claims.

## Claims

1. A system to detect a breach of an outer insulative sheath (122S) in a bipolar electrosurgical instrument (102) that includes, an end effector (108); a conductive lead (122-1) to electrically couple the end effector (108) to an electrosurgical generator unit (106); and an electrically conductive shield (212) surrounding the conductive lead (122-1); wherein the outer insulating sheath (122S) surrounds the conductive shield (212), the system comprising:
a first pulse detection circuit (502-1, 504-1, 506-1, 508-1, 510-1, 512-1, 514-1) to detect a first high frequency (HF) signal component of an HF signal conducted on the conductive lead (122-1);
a second pulse detection circuit (502-3, 504-3, 506-3, 508-3, 510-3, 512-3, 514-3) to detect a second HF signal component of the HF signal conducted on the conductive shield (212);
phase difference sampling logic (516, 610) to produce a succession of sample values indicative of a phase difference over a time interval between the first HF signal component and the second HF signal component;
current detection logic (516, 612) to detect aberrant current flow between the conductive shield (212) and anatomical tissue (130) based upon the succession of sample values indicating that the first HF signal component and the second HF signal component have phases that differ by at least a threshold amount.

2. The system of claim 1,
wherein the first pulse detection circuit includes a first comparator circuit (508-1) to compare the first HF signal component to a first threshold; and
wherein the second pulse detection circuit includes a second comparator circuit (508-3) to compare the second HF signal component to a second threshold.

3. The system of claim 2 further including:
a first threshold level setting circuit (542) to set the first threshold; and
a second threshold setting circuit (544) to set the second threshold.

4. The system of claim 2,
wherein the first pulse detection circuit further includes at least one first low pass filter (510-1); and
wherein the second pulse detection circuit further includes at least one second low pass filter (510-3).

5. The system of claim 2,
wherein the first pulse detection circuit further includes at least one first noise filter (512-1); and
wherein the second pulse detection circuit further includes at least one second noise filter (512-2).

6. The system of claim 1 further including:
a first coupling capacitor (406) to couple the first HF signal component to the first pulse detection circuit; and
a second coupling capacitor (424) to couple the second HF signal component to the second pulse detection circuit.

7. The system of claim 1,
wherein the current detection logic (516) detects current flow based upon a count of high phase angle event samples.

8. The system of claim 1,
wherein the current detection logic (516) detects current flow based upon a comparison of a count of high phase angle event samples and a count of low phase angle event sample pulses.

9. The system of claim 1,
wherein the current detection logic detects current flow in response to an occurrence of a predetermined number of consecutive high phase angle event samples.

10. The system of claim 1,
wherein the current detection logic (516) detects current flow in response to an occurrence of a predetermined ratio of high phase angle event samples and low phase angle event samples during a predetermined time duration.

11. A method to detect a breach of an outer insulative sheath (122S) in a bipolar electrosurgical instrument (102) that includes, an end effector (108); a conductive lead (122-1) to electrically couple the end effector (108) to an electrosurgical generator unit (106); and an electrically conductive shield (212) surrounding the conductive lead (122); wherein the outer insulating sheath (122S) surrounds the conductive shield (212), the method comprising:
detecting pulses of a first HF signal component of an HF signal conducted on the conductive lead (122-1) by the first pulse detection circuit (502-1, 504-1, 506-1, 508-1, 510-1, 512-1, 514-1);
detecting pulses of a second HF signal component of the HF signal conducted on the conductive shield (212) by the second pulse detection circuit (502-3, 504-3, 506-3, 508-3, 510-3, 512-3, 514-3);
producing a succession of sample values, by phase difference sampling logic (516, 610), indicative of phase difference over a time interval between the pulses of the first HF signal component and the second HF signal component;
detecting aberrant current flow, by current detection logic (516, 612), between the conductive shield (212) and anatomical tissue (130) based upon the succession of sample values indicating that the first HF signal component and the second HF signal component have phases that differ by at least a threshold amount.

12. The method of claim 11,
wherein detecting pulses of the first HF signal component includes comparing the first HF signal component to a first threshold; and
wherein detecting pulses of the second HF signal component includes comparing the second HF signal component to a second threshold.

13. The method of claim 11,
wherein detecting current flow between the conductive shield and anatomical tissue based upon the succession of sample values includes detecting based upon a count of high phase angle event samples.

14. The method of claim 11,
wherein detecting current flow between the conductive shield and anatomical tissue based upon the succession of sample values includes detecting based upon a comparison of a count of high phase angle event samples and a count of low phase angle event sample pulses.

15. The method of claim 11,
wherein detecting current flow between the conductive shield (212) and anatomical tissue (130) based upon the succession of sample values includes detecting in response to an occurrence of a predetermined number of consecutive high phase angle event samples.

## Patentansprüche

1. System zur Erkennung eines Bruchs einer äußeren isolierenden Hülle (122S) in einem bipolaren elektrochirurgischen Instrument (102), das einen Endeffektor (108); eine leitende Ader (122-1), um den Endeffektor (108) elektrisch an eine elektrochirurgische Generatoreinheit (106) zu koppeln; und eine elektrisch leitende Abschirmung (212) einschließt, die die leitende Ader (122-1) umgibt;
wobei die äußere isolierende Hülle (122S) die leitende Abschirmung (212) umgibt, wobei das System umfasst:
eine erste Impulserkennungsschaltung (502-1, 504-1, 506-1, 508-1, 510-1, 512-1, 514-1), um eine erste Hochfrequenzsignalkomponente (HF-Signalkomponente) eines HF-Signals zu erkennen, die auf der leitenden Ader (122-1) geführt wird;
eine zweite Impulserkennungsschaltung (502-3, 504-3, 506-3, 508-3, 510-3, 512-3, 514-3), um eine zweite HF-Signalkomponente des HF-Signals zu erkennen, die auf der leitenden Abschirmung (212) geführt wird;
Phasendifferenz-Abtastlogik (516, 610), um eine Folge von Abtastwerten zu produzieren, die eine Phasendifferenz zwischen der ersten HF-Signalkomponente und der zweiten HF-Signalkomponente über ein Zeitintervall angeben;
Stromerkennungslogik (516, 612), um einen abweichenden Stromfluss zwischen der leitenden Abschirmung (212) und anatomischem Gewebe (130) auf der Basis der Folge von Abtastwerten zu erkennen, der angibt, dass die erste HF-Signalkomponente und die zweite HF-Signalkomponente Phasen aufweisen, die sich um mindestens einen Schwellenwertbetrag unterscheiden.

2. System nach Anspruch 1,
wobei die erste Impulserkennungsschaltung eine erste Komparatorschaltung (508-1) einschließt, um die erste HF-Signalkomponente mit einem ersten Schwellenwert zu vergleichen; und
wobei die zweite Impulserkennungsschaltung eine zweite Komparatorschaltung (508-3) einschließt, um die zweite HF-Signalkomponente mit einem zweiten Schwellenwert zu vergleichen.

3. System nach Anspruch 2, ferner einschließend:
eine erste Schwellenwertpegeleinstellungsschaltung (542), um den ersten Schwellenwert einzustellen; und
eine zweite Schwellenwerteinstellungsschaltung (544), um den zweiten Schwellenwert einzustellen.

4. System nach Anspruch 2,
wobei die erste Impulserkennungsschaltung ferner mindestens einen ersten Tiefpassfilter (510-1) einschließt; und
wobei die zweite Impulserkennungsschaltung ferner mindestens einen zweiten Tiefpassfilter (510-3) einschließt.

5. System nach Anspruch 2,
wobei die erste Impulserkennungsschaltung ferner mindestens einen ersten Rauschfilter (512-1) einschließt; und
wobei die zweite Impulserkennungsschaltung ferner mindestens einen zweiten Rauschfilter (512-2) einschließt.

6. System nach Anspruch 1, ferner einschließend:
einen ersten Koppelkondensator (406), um die erste HF-Signalkomponente an die erste Impulserkennungsschaltung zu koppeln; und
einen zweiten Koppelkondensator (424), um die zweite HF-Signalkomponente an die zweite Impulserkennungsschaltung zu koppeln.

7. System nach Anspruch 1,
wobei die Stromerkennungslogik (516) einen Stromfluss auf der Basis eines Zählwerts von Abtastungen von Ereignissen mit hohem Phasenwinkel erkennt.

8. System nach Anspruch 1,
wobei die Stromerkennungslogik (516) einen Stromfluss auf der Basis eines Vergleichs eines Zählwerts von Abtastungen von Ereignissen mit hohem Phasenwinkel und eines Zählwerts von Abtastimpulsen von Ereignissen mit niedrigem Phasenwinkel erkennt.

9. System nach Anspruch 1,
wobei die Stromerkennungslogik einen Stromfluss als Reaktion auf ein Eintreten einer vorbestimmten Anzahl von aufeinanderfolgenden Abtastungen von Ereignissen mit hohem Phasenwinkel erkennt.

10. System nach Anspruch 1,
wobei die Stromerkennungslogik (516) einen Stromfluss als Reaktion auf ein Eintreten eines vorbestimmten Verhältnisses von Abtastungen von Ereignissen mit hohem Phasenwinkel und Abtastungen von Ereignissen mit niedrigem Phasenwinkel während einer vorbestimmten Zeitdauer erkennt.

11. Verfahren zur Erkennung eines Bruchs einer äußeren isolierenden Hülle (122S) in einem bipolaren elektrochirurgischen Instrument (102), das einen Endeffektor (108); eine leitende Ader (122-1), um den Endeffektor (108) elektrisch an eine elektrochirurgische Generatoreinheit (106) zu koppeln; und eine elektrisch leitende Abschirmung (212) einschließt, die die leitende Ader (122) umgibt; wobei die äußere isolierende Hülle (122S) die leitende Abschirmung (212) umgibt, wobei das Verfahren umfasst:
Erkennen von Impulsen einer ersten HF-Signalkomponente eines HF-Signals, die auf der leitenden Ader (122-1) geführt wird, durch die erste Impulserkennungsschaltung (502-1, 504-1, 506-1, 508-1, 510-1, 512-1, 514-1);
Erkennen von Impulsen einer zweiten HF-Signalkomponente des HF-Signals, die auf der leitenden Abschirmung (212) geführt wird, durch die zweite Impulserkennungsschaltung (502-3, 504-3, 506-3, 508-3, 510-3, 512-3, 514-3);
Produzieren einer Folge von Abtastwerten durch eine Phasendifferenz-Abtastlogik (516, 610), die eine Phasendifferenz zwischen den Impulsen der ersten HF-Signalkomponente und der zweiten HF-Signalkomponente über ein Zeitintervall angeben;
Erkennen eines abweichenden Stromflusses zwischen der leitenden Abschirmung (212) und anatomischem Gewebe (130) durch eine Stromerkennungslogik (516, 612) auf der Basis der Folge von Abtastwerten, der angibt, dass die erste HF-Signalkomponente und die zweite HF-Signalkomponente Phasen aufweisen, die sich um mindestens einen Schwellenwertbetrag unterscheiden.

12. Verfahren nach Anspruch 11,
wobei das Erkennen von Impulsen der ersten HF-Signalkomponente ein Vergleichen der ersten HF-Signalkomponente mit einem ersten Schwellenwert einschließt; und
wobei das Erkennen von Impulsen der zweiten HF-Signalkomponente ein Vergleichen der zweiten HF-Signalkomponente mit einem zweiten Schwellenwert einschließt.

13. Verfahren nach Anspruch 11,
wobei das Erkennen eines Stromflusses zwischen der leitenden Abschirmung und anatomischem Gewebe auf der Basis der Folge von Abtastwerten ein Erkennen auf der Basis eines Zählwerts von Abtastungen von Ereignissen mit hohem Phasenwinkel einschließt.

14. Verfahren nach Anspruch 11,
wobei das Erkennen eines Stromflusses zwischen der leitenden Abschirmung und anatomischem Gewebe auf der Basis der Folge von Abtastwerten ein Erkennen auf der Basis eines Vergleichs eines Zählwerts von Abtastungen von Ereignissen mit hohem Phasenwinkel und eines Zählwerts von Abtastimpulsen von Ereignissen mit niedrigem Phasenwinkel einschließt.

15. Verfahren nach Anspruch 11,
wobei das Erkennen eines Stromflusses zwischen der leitenden Abschirmung (212) und anatomischem Gewebe (130) auf der Basis der Folge von Abtastwerten ein Erkennen als Reaktion auf ein Eintreten einer vorbestimmten Anzahl von aufeinanderfolgenden Abtastungen von Ereignissen mit hohem Phasenwinkel einschließt.

## Revendications

1. Système pour détecter une rupture d'une gaine isolante externe (122S) dans un instrument électrochirurgical bipolaire (102) qui inclut un effecteur terminal (108) ; un fil conducteur (122-1) pour coupler électriquement l'effecteur terminal (108) à une unité génératrice électrochirurgicale (106) ; et un blindage conducteur électriquement (212) entourant le fil conducteur (122-1) ;
dans lequel la gaine isolante externe(122S) entoure le blindage conducteur (212), le système comprenant :
un premier circuit de détection d'impulsions (502-1, 504-1, 506-1, 508-1, 510-1, 512-1, 514-1) pour détecter une première composante de signal haute fréquence (HF) d'un signal HF conduit sur le fil conducteur (122-1) ;
un deuxième circuit de détection d'impulsions (502-3, 504-3, 506-3, 508-3, 510-3, 512-3, 514-3) pour détecter une deuxième composante de signal HF du signal HF conduit sur le blindage conducteur (212) ;
une logique d'échantillonnage de différence de phase (516, 610) pour produire une succession de valeurs d'échantillon indiquant une différence de phase sur un intervalle de temps entre la première composante de signal HF et la deuxième composante de signal HF ;
une logique de détection de courant (516, 612) permettant de détecter un flux de courant aberrant entre le blindage conducteur (212) et un tissu anatomique (130) sur la base de la succession de valeurs d'échantillon indiquant que la première composante de signal HF et la deuxième composante de signal HF ont des phases qui diffèrent d'au moins une valeur seuil.

2. Système selon la revendication 1,
dans lequel le premier circuit de détection d'impulsions inclut un premier circuit comparateur (508-1) pour comparer la première composante de signal HF à un premier seuil ; et
dans lequel le deuxième circuit de détection d'impulsions inclut un deuxième circuit comparateur (508-3) pour comparer la deuxième composante de signal HF à un deuxième seuil.

3. Procédé selon la revendication 2 incluant en outre :
un premier circuit de réglage de niveau de seuil (542) pour régler le premier seuil ; et
un deuxième circuit de réglage de seuil (544) pour régler le deuxième seuil.

4. Système selon la revendication 2,
dans lequel le premier circuit de détection d'impulsions inclut en outre au moins un premier filtre passe-bas (510-1) ; et
dans lequel le deuxième circuit de détection d'impulsions inclut en outre au moins un deuxième filtre passe-bas (510-3).

5. Système selon la revendication 2,
dans lequel le premier circuit de détection d'impulsions inclut en outre au moins un premier filtre de bruit (512-1) ; et
dans lequel le deuxième circuit de détection d'impulsions inclut en outre au moins un deuxième filtre de bruit (512-2).

6. Système selon la revendication 1 incluant en outre :
un premier condensateur de couplage (406) pour coupler la première composante de signal HF au premier circuit de détection d'impulsions ; et
un deuxième condensateur de couplage (424) pour coupler la deuxième composante de signal HF au deuxième circuit de détection d'impulsions.

7. Système selon la revendication 1,
dans lequel la logique de détection de courant (516) détecte un flux de courant sur la base d'un comptage d'échantillons d'événements à angle de phase élevé.

8. Système selon la revendication 1,
dans lequel la logique de détection de courant (516) détecte un flux de courant sur la base d'une comparaison d'un comptage d'échantillons d'événements à angle de phase élevé et d'un comptage d'impulsions d'échantillons d'événements à angle de phase faible.

9. Système selon la revendication 1,
dans lequel la logique de détection de courant détecte un flux de courant en réponse à une apparition d'un nombre prédéterminé d'échantillons consécutifs d'événements à angle de phase élevé.

10. Système selon la revendication 1,
dans lequel la logique de détection de courant (516) détecte un flux de courant en réponse à une apparition d'un rapport prédéterminé d'échantillons d'événements à angle de phase élevé et d'échantillons d'événements à angle de phase faible pendant une durée prédéterminée.

11. Procédé pour détecter une rupture d'une gaine isolante externe (122S) dans un instrument électrochirurgical bipolaire (102) qui inclut, un effecteur terminal (108) ; un fil conducteur (122-1) pour coupler électriquement l'effecteur terminal (108) à une unité génératrice électrochirurgicale (106) ; et un blindage connducteur électriquement (212) entourant le fil conducteur (122) ; dans lequel la gaine isolante externe (122S) entoure le blindage conducteur (212), le procédé comprenant :
la détection d'impulsions d'une première composante de signal HF d'un signal HF conduit sur le fil conducteur (122-1) par le premier circuit de détection d'impulsions (502-1, 504-1, 506-1, 508-1, 510-1, 512-1, 514-1) ;
la détection d'impulsions d'une deuxième composante de signal HF du signal HF conduit sur le blindage conducteur (212) par le deuxième circuit de détection d'impulsions (502-3, 504-3, 506-3, 508-3, 510-3, 512-3, 514-3) ;
la production d'une succession de valeurs d'échantillon, par une logique d'échantillonnage de différence de phase (516, 610), indiquant une différence de phase sur un intervalle de temps entre les impulsions de la première composante de signal HF et la deuxième composante de signal HF ;
la détection d'un flux de courant aberrant, par une logique de détection de courant (516, 612), entre le blindage conducteur (212) et le tissu anatomique (130) sur la base de la succession de valeurs d'échantillon indiquant que la première composante de signal HF et la deuxième composante de signal HF ont des phases qui diffèrent d'au moins une valeur seuil.

12. Procédé selon la revendication 11,
dans lequel la détection d'impulsions de la première composante de signal HF inclut la comparaison de la première composante de signal HF à un premier seuil ; et
la détection d'impulsions de la deuxième composante de signal HF inclut la comparaison de la deuxième composante de signal HF à un deuxième seuil.

13. Procédé selon la revendication 11,
dans lequel la détection d'un flux de courant entre le blindage conducteur et un tissu anatomique sur la base de la succession de valeurs d'échantillon inclut la détection sur la base d'un comptage d'échantillons d'événements à angle de phase élevé.

14. Procédé selon la revendication 11,
dans lequel la détection d'un flux de courant entre le blindage conducteur et le tissu anatomique sur la base de la succession de valeurs d'échantillon inclut la détection sur la base d'une comparaison d'un comptage d'échantillons d'événements à angle de phase élevé et d'un comptage d'impulsions d'échantillons d'événements à angle de phase faible.

15. Procédé selon la revendication 11,
dans lequel la détection d'un flux de courant entre le blindage conducteur (212) et un tissu anatomique (130) sur la base de la succession de valeurs d'échantillon inclut la détection en réponse à l'apparition d'un nombre prédéterminé d'échantillons consécutifs d'événements à angle de phase élevé.
